# EUROPÄISCHE PATENTANMELDUNG

(11) **EP 2 078 492 A1**
(43) Veröffentlichungstag der Anmeldung: **15.07.2009**
(21) Anmeldenummer: 08000435.1
(22) Anmeldetag: 11.01.2008
(51) Int. Cl.: A61B 5/00, A61B 5/151, G01N 33/487, G01N 35/00

(54) **Bandkassette für ein medizinisches Handgerät und Blutzuckermesssystem**

(71) Anmelder: Roche Diagnostics GmbH, 68305 Mannheim (DE); F.HOFFMANN-LA ROCHE AG, 4070 Basel (CH)
(72) Erfinder: Harttig, Herbert, Dr., 67434 Neustadt (DE)
(74) Vertreter: Mommer, Niels

(57) **Zusammenfassung**

Bandkassette für ein medizinisches Handgerät (1), wobei die Bandkassette (5) ein Trägerband (6) enthält, das als Funktionselemente Testfelder (8) zur Untersuchung einer Probe einer menschlichen oder tierischen Körperflüssigkeit und/oder Lanzetten (7) trägt, wobei das Trägerband (6) bei Gebrauch an Reibungsstellen (12,14) der Bandkassette (5) vorbei gleitet.
Es ist vorgesehen, dass zur Reibungsreduktion mindestens eine der Reibungsstellen (14,12) eine Oberfläche aus einem Fluorpolymer aufweist.
Gezeigt ist ferner ein Blutzuckermesssystem mit einem Handgerät (1), das ein Trägerband (6), das als Funktionselemente Testfelder (8) zur Untersuchung einer Probe einer Körperflüssigkeitsprobe und/oder Lanzetten (7) trägt, und einen Transportmechanismus enthält, mit dem die Funktionselemente (7,8) nach einander in eine Gebrauchsposition bewegt werden können, wobei das Trägerband (6) an mindestens einer Reibungsstelle (14,12) mit einer Oberfläche aus einem Fluorpolymer vorbei gleitet.

## Beschreibung

Die Erfindung betrifft eine Bandkassette mit den im Oberbegriff des Anspruchs 1 angegebenen Merkmalen.

Eine derartige Bandkassette ist aus der EP 1 424 040 A1 bekannt. Das Trägerband der bekannten Bandkassette trägt als Funktionselemente Testfelder zur Untersuchung einer Körperflüssigkeitsprobe und wird zum Gebrauch in ein Messgerät eingesetzt, mit dem das Vorhandensein oder die Konzentration eines Analyten, insbesondere die Glukosekonzentration, bestimmt werden. Solche Bandkassetten werden insbesondere von Diabetikern benötigt, die mehrmals täglich eine Messung der Glucosekonzentration einer durch einen Lanzettenstich gewonnenen Körperflüssigkeitsprobe, in der Regel Blut oder interstitielle Flüssigkeit, durchführen müssen.

Bekannt sind auch Bandkassetten mit einem Trägerband, das als Funktionselemente sowohl Testfelder als auch Lanzetten trägt. Derartige Bandkassetten sind für integrierte Handgeräte bestimmt, mit denen sowohl eine Messung einer Körperflüssigkeitsprobe als auch ein Lanzettenstich zur Probengewinnung vorgenommen werden können. Einfachere Blutzuckermesssysteme umfassen jedoch häufig zwei separate Handgeräte, nämlich ein Stechgerät und ein Messgerät. Für derartige Stechgeräte sind Bandkassetten bekannt, deren Trägerbänder als Funktionselemente Lanzetten tragen.

Für einen hohen Benutzerkomfort ist bei medizinischen Handgeräten, wie sie insbesondere von Diabetikern verwendet werden, ein möglichst leichter Bandtransport wichtig. Bein einfacheren Handgeräten wird der Bandtransport manuell von dem Benutzer bewirkt, komplexere Handgeräte haben einen Elektromotor, um die Funktionselemente des Trägerbandes nacheinander in eine Gebrauchsposition zu bringen.

Aufgabe der Erfindung ist es deshalb, einen Weg aufzuzeigen, wie bei einem medizinischen Handgerät der Transport eines Trägerbandes erleichtert werden kann.

Diese Aufgabe wird durch eine Bandkassette mit den im Anspruch 1 angegebenen Merkmalen gelöst. Vorteilhafte Weiterbildungen der Erfindung sind Gegenstand der Unteransprüche. Die Aufgabe wird auch gelöst durch ein Blutzuckermesssystem mit den im Anspruch 10 angegebenen Merkmalen.

Erfindungsgemäß hat zur Reibungsreduktion mindestens eine der Reibungsstellen, an denen das Trägerband bei Gebrauch vorbeigleitet, eine Oberfläche aus einem Fluorpolymer. Überraschenderweise lässt sich durch diese einfache Maßnahme die Bandreibung wesentlich reduzieren.

Anstatt eine Reduktion der Reibungskraft durch eine geänderte Bandführung mit einem reduzierten Umschlingungswinkel und somit einer geringeren Reibung anzustreben, oder die Andruckkraft, mit der das Trägerband auf den Reibungsstellen lastet, zu reduzieren oder das Material des Trägerbandes hinsichtlich seiner Reibungseigenschaften zu optimieren, wurde mit der vorliegenden Erfindung ein völlig anderer Weg eingeschlagen. Die Oberflächen von Reibungsstellen einer Bandkassette oder eines medizinischen Handgeräts, in welches eine Bandkassette bestimmungsgemäß eingelegt wird, lassen sich mit Fluorpolymeren vorteilhaft reibungsarm ausgestalten. Beispielsweise kann das Fluorpolymer als eine Beschichtung aufgetragen werden, insbesondere als Lack oder Folie. Möglich ist es aber auch, das entsprechende Bauteil vollständig aus Fluorpolymer herzustellen oder Fluorpolymer zusammen mit einem anderen Kunststoff in einem Bi-Komponenten-Spritzgussverfahren zu verarbeiten.

Die Erfindung hat den Vorteil, dass sich auch ohne eine Änderung der Bandführung oder des mechanischen Transportmechanismus mit verhältnismäßig einfachen Mitteln eine erhebliche Reduktion der Bandreibung erzielen lässt. Bei manuell betriebenen Handgeräten muss ein Benutzer deshalb für den Bandtransport nur noch eine wesentlich geringere Kraft aufbringen, was den Benutzerkomfort erhöht. Bei Geräten mit einem Elektromotor kann ein leistungsschwächerer und folglich kleinerer sowie kostengünstigerer Motor verwendet werden. Hinzu kommt, dass der Energieverbrauch des Motors reduziert ist, so dass kleinere, leichtere und kostengünstigere Batterien ausreichen.

Als Fluorpolymere im Sinne der Erfindung werden alle organischen und siliziumorganischen Polymere verstanden die Fluor in kovalenter Bindung in ihren Monomeren enthalten und in reiner Form oder als Mischung oder Legierung oder als Copolymere mit anderen nicht fluorhaltigen Polymeren vorliegen. Das für die vorliegende Erfindung verwendete Fluorpolymer kann beispielsweise Polytetrafluorethylen (PTFE) oder Polyvinylidenfluorid (PVDF) enthalten, bevorzugt mindestens 10 Gew. %, beispielsweise 10 Gew. % bis 40 Gew. %. Besonders bevorzugt als Fluorpolymer ist eine Mischung aus Polypropylen und PTFE.

Testfelder zum Untersuchen einer Probe einer menschlichen oder tierischen Körperflüssigkeit enthalten in der Regel empfindliche Nachweisreagenzien, beispielsweise zur photometrischen Bestimmung einer Analytkonzentration. Bandkassetten mit einem Trägerband, das als Funktionselemente Testfelder zum Untersuchen einer Körperflüssigkeitsprobe trägt, müssen deshalb in der Regel ein abgedichtetes Gehäuse haben, damit unbenutzte Testfelder nicht durch Feuchtigkeit beeinträchtigt werden. Eine mit einer Dichtung versehene Bandaustrittsöffnung ist bei derartigen Kassetten eine erhebliche Reibungsquelle. Die Erfindung kann deshalb besonders vorteilhaft für eine Reibungsstelle genutzt werden, die sich an einer Bandaustrittsöffnung eines Kassettengehäuses befindet. Beispielsweise kann eine Bandaustrittsöffnung mit einer Dichtlippe, insbesondere mir einer elastomeren Dichtlippe, abgedichtet sein, die eine Oberfläche aus einem Fluorpolymer aufweist. Die Oberfläche aus Fluorpolymer kann als Lack aufgetragen sein. Möglich ist es auch, ein fluorpolymerhaltiges Elastomer als Material für die Dichtlippe zu verwenden.

Weitere Einzelheiten und Vorteile der Erfindung werden an Ausführungen unter Bezugnahme auf die beigefügten Zeichnungen erläutert. Die dabei beschriebenen Merkmale können einzeln oder in Kombination zum Gegenstand von Ansprüchen gemacht werden. Es zeigen:
- Figur 1:: ein Ausführungsbeispiel eines Handgeräts zur Messung der Glukosekonzentration;
- Figur 2:: eine Bandkassette für das in Figur 1 gezeigte Handgerät;
- Figur 3:: eine Detailansicht eines Ausführungsbeispiels einer Bandaustrittsöffnung einer Bandkassette;
- Figur 4:: ein weiteres Ausführungsbeispiel einer Bandaustrittsöffnung; und
- Figur 5:: eine Prinzipskizze eine Mechanismus zur Reduktion der Bandreibung an einer Bandaustrittsöffnung einer Bandkassette.

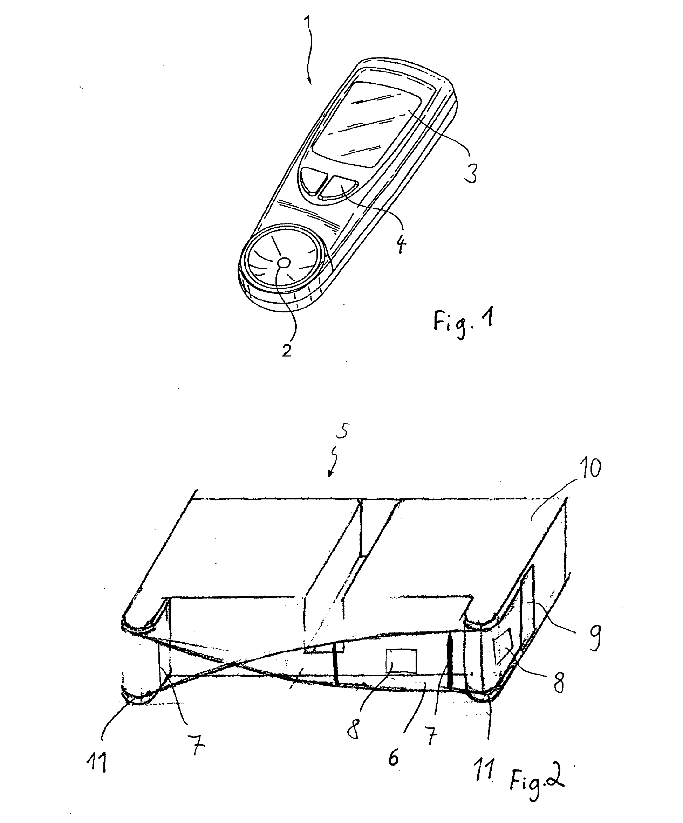

Figur 1 zeigt ein Ausführungsbeispiel eines medizinischen Handgeräts 1 zur Bestimmung einer Analytkonzentration einer menschlichen Körperflüssigkeitsprobe. Derartige Handgeräte 1 werden insbesondere von Diabetikern zur Bestimmung der Glukosekonzentration einer Probe von Blut oder interstitieller Flüssigkeit verwendet.

Das Handgerät 1 hat eine Geräteöffnung 2, gegen die ein Körperteil zur Entnahme einer Körperflüssigkeitsprobe gepresst wird. Ein Messresultat wird mit einer Anzeigeeinrichtung 3, beispielsweise einer Flüssigkristallanzeige, angezeigt. Zur Bedienung des Messgeräts 1 sind Bedienungselemente 4 in Form von Tasten vorgesehen.

Das in Figur 1 gezeigte Messgerät 1 hat an seiner Rückseite ein Fach, in das eine in Figur 2 dargestellte Bandkassette 5 eingelegt werden kann. Das Messgerät 1 und die Bandkassette 5 bilden zusammen ein Blutzuckermesssystem. Die Bandkassette 5 enthält ein Trägerband 6, das als Funktionselemente Lanzetten 7 zur Gewinnung einer Körperflüssigkeitsprobe durch einen Einstich und Testfelder 8 zur Untersuchung einer Körperflüssigkeitsprobe trägt. Das Messgerät 1 enthält eine nicht dargestellte Bandfördereinrichtung, um die Funktionselemente 7, 8 nacheinander in eine Gebrauchsposition zu bringen, so dass mit den Lanzetten 7 in ein an die Geräteöffnung 2 angelegtes Körperteil gestochen und mit einem Testfeld 8 eine aus der erzeugten Stichwunde austretende Körperflüssigkeitsprobe aufgenommen und photometrisch untersucht werden kann. Die Testfelder 8 enthalten Nachweisreagenzien, die bei Kontakt mit einer Probe eine konzentrationsabhängige Verfärbung des Testfelds 8 bewirken. Diese Verfärbung kann mit einer in dem Messgerät 1 enthaltenen Messeinheit gemessen und ausgewertet werden.

Beim Bandtransport gleitet das Trägerband 6, das beispielsweise aus Polyester sein kann, an verschiedenen Reibungsstellen der Bandkassette 5 und in der Regel auch des Messgeräts 1 vorbei. Bei dem in Figur 2 dargestellten Ausführungsbeispiel ergeben sich Reibungsstellen beispielsweise an einer Bandaustritts- und einer Bandeintrittsöffnung 9 des Kassettengehäuses 10 sowie an Bandführungselementen 11, an denen das Trägerband 6 entlang gleitet. Zur Reibungsreduktion haben die Reibungsstellen des dargestellten Ausführungsbeispiels eine Oberfläche aus einem Fluorpolymer. Das Fluorpolymer kann beispielsweise eine Beschichtung sein, insbesondere ein Lack oder eine Folie. Möglich ist es auch, dass das Fluorpolymer in einem Bi-Komponenten-Spritzgussverfahren beim Herstellen des Kassettengehäuses aufgebracht wurde.

Bei dem dargestellten Ausführungsbeispiel ist das Trägerband 6 zwischen seinem Austritt aus dem Kassettengehäuse 10 und seinem Eintritt um 180° verdreht. Auf diese Weise kann die Orientierung der Lanzetten 7 für einen Stich in Bezug auf die Geräteöffnung 2 ausgerichtet werden. Möglich ist es aber auch, das Trägerband 6 verdrehungsfrei zu führen.

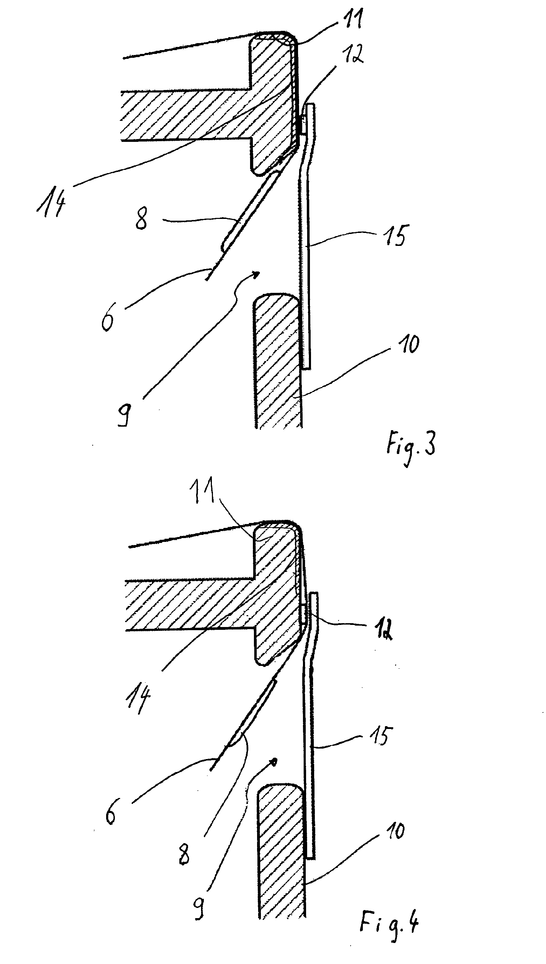

Ein wesentlicher Teil der Bandreibung entsteht im Bereich von Öffnungen 9 des Kassettengehäuses 10, durch welche das Trägerband 6 hindurchgeführt ist. Da die Testfelder 8 feuchtigkeitsempfindlich sind, ist das Trägerband 6 oder zumindest der Bandabschnitt mit unbenutzten Funktionselementen 8 in einem abgedichteten Gehäuse oder einem abgedichteten Teil des Kassettengehäuses 10 angeordnet und die Bandaustrittsöffnung 9 deshalb mit einem in Figur 3 gezeigten Dichtelement 12 versehen, das bevorzugt als eine Dichtlippe aus einem elastomeren Material gebildet ist. Derartige Dichtelemente können insbesondere beim Vorbeiführen der Funktionselemente 7, 8 zu einer sehr hohen Reibung führen. Zur Reibungsreduktion hat das Dichtelement 12 deshalb bei dem dargestellten Ausführungsbeispiel eine Oberfläche aus einem Fluorpolymer. Das Fluorpolymer kann als eine Beschichtung, insbesondere als Lack, aufgetragen sein. Möglich ist es auch, dass das Dichtelement 12 aus einem elastomeren Fluorpolymer hergestellt ist.

In Figur 3 ist eine Detailansicht der Bandaustrittsöffnung 9 der Bandkassette 1 dargestellt. Zur Reibungsreduktion sind die Testfelder 8 auf der von dem Dichtelement 12 abgewandten Seite des Trägerbandes 6 angeordnet. Auf diese Weise können die verhältnismäßig rauen Testfelder 8 an einer von dem Kassettengehäuse 10 gebildeten Reibungsstelle 14 entlang gleiten, die eine Oberfläche aus Fluorpolymer aufweist, während die relativ glatte Bandrückseite an der von dem Dichtelement 12 gebildeten Reibungsstelle entlang gleitet. Das Dichtelement 12 ist an einem Deckelement 15, beispielsweise einer Folie befestigt, welche die Bandaustrittsöffnung 9 bedeckt. Das Deckelement 15, beispielsweise aus Aluminium, Kunststoff oder einem Verbund, wird durch ein nicht gezeigtes Federelement 19 belastet und drückt das an seinem Ende angeordnete Dichtelement 14 federnd gegen das Trägerband 6.

Die Reibungsstelle 14 des Kassettengehäuses 10 kann beispielsweise in einem Bikomponentenspritzgussverfahren mit einer Fluorpolymeroberfläche versehen werden. Das Bikomponentenspritzgussverfahren kann beispielsweise durchgeführt werden, indem in eine Spritzgussform für ein Kassettengehäuse 10 ein Wechselkern eingesetzt wird, der Kavitäten für die Ausformung der Reibungsstellen bildet. Die Kavitäten werden im Spritzguss gefüllt, wobei eine Mischung aus Polypropylen mit 20 Gew.% PTFE Mikropulver verwendet werden kann, das beispielsweise von der Fa. Dyneon GmbH & Co. KG, Neuss, Deutschland erhältlich ist. Für einen zweiten Schuss wird der Wechselkern ausgetauscht und das Gehäuse mit Polypropylen ohne PTFE Anteil fertig gespritzt.

Die Reibungsstelle 14 kann beispielsweise auch durch Hinterspritzen einer PTFE Folie erzeugt werden.

Wird die Fluorpolymeroberfläche als Lack aufgetragen, ist es vorteilhaft, das Kassettengehäuse 10, beispielsweise ein Polypropylengehäuse, durch Plasmabehandlung, insbesondere atmosphärischem Plasma oder einer anderen geeigneten Aktivierungsmethode, vorzubehandeln, um eine bessere Haftung des Lacks zu erzielen. Bevorzugt für die Reibungsstellen des Gehäuses oder des Dichtelements 12 sind PTFE-haltige Lacke. Geeignete Lacke werden beispielsweise von der Firma Fuchs Lubritech, Weilerbach, Deutschland, unter der Bezeichnung gleitmo SFL9062K und gleitmo SFL9460K1 vertrieben.

Ein weiteres Ausführungsbeispiel einer Bandeintritts- oder Bandaustrittsöffnung 9 einer Bandkassette 5 ist in Figur 4 gezeigt, dieses Ausführungsbeispiel unterscheidet sich von dem in Figur 3 gezeigten Ausführungsbeispiel darin, dass das Dichtelement nicht an dem Deckelement 15, sondern an dem Kassettengehäuse 10 befestigt ist. Die Funktionselemente 7, 8 sind deshalb auf der anderen Seite des Trägerbandes 6 angeordnet, so dass diese dem Deckelement 15 zugewandt sind. Da bei diesem Ausführungsbeispiel das Deckelement 15 an dem Trägerband 6 anliegt und deshalb eine Reibungsstelle ausbildet, hat das Deckelement 15 ebenfalls eine Oberfläche aus einem Fluorpolymer. Beispielsweise kann das Deckelement 15 eine Aluminiumfolie sein, auf die Fluorpolymer als Lack aufgebracht wurde, beispielsweise ein PTFE-haltiger Lack. Zur besseren Haftung des Lacks kann das Deckelement 15 vor dessen Auftragen einer Aktivierung, z. B. einer Plasmabehandlung, unterzogen werden.

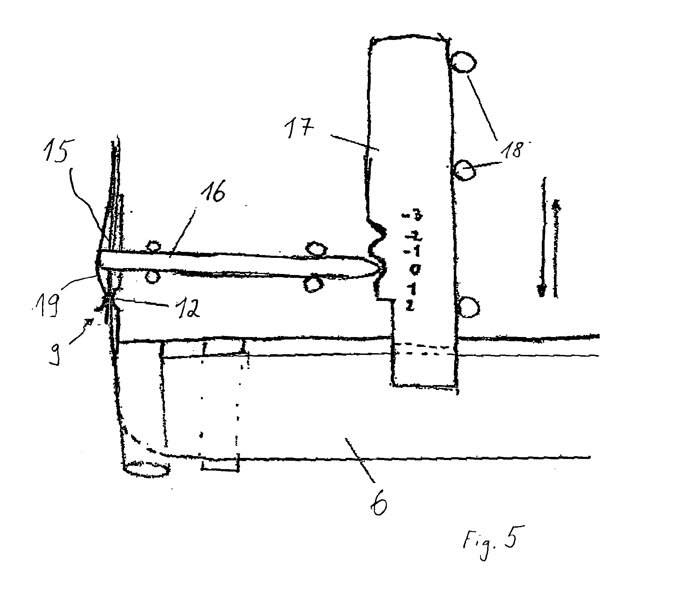

Figur 5 zeigt eine Prinzipskizze eines Mechanismus, mit dem eine zusätzliche Reduktion der Bandreibung an der Bandaustrittsöffnung 9 einer Bandkassette 5 erreicht werden kann. Der in Figur 5 gezeigte Mechanismus bewirkt, dass die Andruckkraft, mit der das Trägerband 6 von dem Deckelement 15 gegen die Dichtung 12 der Bandaustrittsöffnung 9 gedrückt wird, beim Bandtransport zeitweise vermindert wird, indem ein mit dem Bandtransportmechanismus gekoppeltes Betätigungselement 16 das die Bandaustrittsöffnung 9 bedeckende Deckelement 15 zeitweise entlastet, indem es ein auf das Deckelement 15 wirkendes Federelement 19 anhebt. Das Betätigungselement 16 kann beispielsweise ein Schieber oder ein Stößel sein, der von einem Aktuator bewegt wird, der bei Bedarf aktiviert wird. Bevorzugt wird das Betätigungselement 16 jedoch von dem Bandtransportmechanismus des Geräts angetrieben, beispielsweise mittels einer Kulissensteuerung. In Figur 5 ist schematisch ein Antriebselement 17 des Messgeräts 1 dargestellt, das in Richtung der in Figur 5 dargestellten Pfeile beweglich ist und dabei von Führungselementen 18 geführt ist. Bei einer solchen Bewegung tastet das Betätigungselement 16 eine Steuerkurve auf der Oberfläche des Antriebselements 17 ab, so dass das Federelement 19 zeitweise angehoben und damit die Bandreibung reduziert wird.

Das Betätigungselement 16 kann mit einer Bandfördereinrichtung, die das Trägerband 6 in seiner Längsrichtung durch Aufwickeln bewegt, gekoppelt sein oder mit einem Transportmechanismus, mit dem das Trägerband 6 quer zu seiner Längsrichtung zu der in Figur 1 dargestellten Geräteöffnung 2 hinbewegt wird, beispielsweise um eine Probe aufzunehmen oder eine Stichwunde zu erzeugen.

### Bezugszahlen

- 1: Handgerät / Messgerät
- 2: Geräteöffnung
- 3: Anzeigeeinrichtung
- 4: Tasten
- 5: Bandkassette
- 6: Trägerband
- 7: Lanzette
- 8: Testfeld
- 9: Bandaustrittsöffnung
- 10: Kassettengehäuse
- 11: Bandführungselement
- 12: Dichtelement

- 14: Reibungsstelle
- 15: Deckelement
- 16: Betätigungselement
- 17: Antriebselement
- 18: Führungselement
- 19: Federelement

## Patentansprüche

1. Bandkassette für ein medizinisches Handgerät (1), wobei die Bandkassette (5) ein Trägerband (6) enthält, das als Funktionselemente Testfelder (8) zur Untersuchung einer Probe einer menschlichen oder tierischen Körperflüssigkeit und/oder Lanzetten (7) trägt, wobei das Trägerband (6) bei Gebrauch an Reibungsstellen (12, 14) der Bandkassette (5) vorbei gleitet,
**dadurch gekennzeichnet, dass**
zur Reibungsreduktion mindestens eine der Reibungsstellen (14, 12) eine Oberfläche aus einem Fluorpolymer aufweist.

2. Bandkassette nach Anspruch 1, **dadurch gekennzeichnet, dass** das Fluorpolymer PTFE oder PVDF enthält.

3. Bandkassette nach einem der vorhergehenden Ansprüche, **dadurch gekennzeichnet, dass** das Fluorpolymer eine Beschichtung ist.

4. Bandkassette nach Anspruch 3, **dadurch gekennzeichnet,** das Fluorpolymer als Lack aufgebraucht ist.

5. Bandkassette nach Anspruch 3, **dadurch gekennzeichnet, dass** das Fluorpolymer als Folie aufgebracht ist.

6. Bandkassette nach einem der Ansprüche 1 bis 3, **dadurch gekennzeichnet, dass** das Fluorpolymer in einem Bikomponentenspritzgussverfahren aufgebracht wurde.

7. Bandkassette nach einem der vorhergehenden Ansprüche, **dadurch gekennzeichnet, dass** sich die Reibungsstelle (14, 12), die eine Oberfläche aus einem Fluorpolymer aufweist, an einer Bandaustrittsöffnung (9) eines Kassettengehäuses (10) befindet.

8. Bandkassette nach Anspruch 7, **dadurch gekennzeichnet, dass** die Reibungsstelle (14, 12), die eine Oberfläche aus einem Fluorpolymer aufweist, von einem Dichtelement (12), insbesondere einer Dichtlippe, gebildet ist.

9. Bandkassette nach Anspruch 7 oder 8, **dadurch gekennzeichnet, dass** die Reibungsstelle von einem die Bandaustrittsöffnung (9) bedeckenden Deckelement (15) gebildet ist.

10. Blutzuckermesssystem mit einem Handgerät (1), das ein Trägerband (6), das als Funktionselemente Testfelder (8) zur Untersuchung einer Probe einer Körperflüssigkeitsprobe und/oder Lanzetten (7) trägt, und einen Transportmechanismus enthält, mit dem die Funktionselemente (7, 8) nach einander in eine Gebrauchsposition bewegt werden können, wobei das Trägerband (6) an Reibungsstellen (14, 12) vorbei gleitet, **dadurch gekennzeichnet, dass** zur Reibungsreduktion mindestens eine der Reibungsstellen (14, 12) eine Oberfläche aus einem Fluorpolymer aufweist.
